# EUROPEAN PATENT APPLICATION

(11) **EP 4 797 730 A1**
(43) Date of publication of application: **26.08.2026**
(21) Application number: 25214249.2
(22) Date of filing: 07.11.2025
(51) Int. Cl.: H04R 25/00, A61B 5/12

(54) **METHOD FOR FITTING HEARING AID AND HEARING AID**

(30) Priority: 11.11.2024 CN 202411604980
(71) Applicant: Anker Innovations Technology Co., Ltd., Changsha, Hunan 410221 (CN)
(72) Inventor: Li, Lei, Changsha City, 410221 (CN)
(74) Representative: Wörz, Volker Alfred

(57) **Abstract**

A method includes: initializing signal-processing parameters of the hearing aid at different frequencies based on attribute data and hearing characteristic data of a user, wherein the signal-processing parameters comprising a gain; processing a hearing test signal to be played based on initialized values of the gain, and obtaining a processed hearing test signal; playing the processed hearing test signal and determining a hearing test result of the user using the hearing aid; and in a case where the hearing test result indicates that the hearing aid does not meet a preset first hear-aiding requirement, adjusting values of the gain of the hearing aid at different frequencies based on the hearing test result, and continuously iterating a hearing test based on the adjusted values of the gain until the hearing aid meets the preset first hear-aiding requirement. A hearing aid is also provided.

## Description

### CROSS-REFERENCE TO RELATED APPLICATIONS

This application claims priority to Chinese Patent Application No. 202411604980.6, filed on November 11, 2024 in the National Intellectual Property Administration of China, the contents of which are herein incorporated by reference in their entireties.

### TECHNICAL FIELD

Embodiments of the present disclosure relate to the field of hearing aid fitting technologies, and in particular to a method for fitting a hearing aid and a hearing aid.

### BACKGROUND

Hearing aids are auditory assistance devices primarily used to help individuals with hearing impairments improve their hearing. The hearing aid enable individuals with hearing impairments to better perceive and understand sounds. Typically, users need to undergo hearing aid fitting before wearing the device to ensure satisfactory results.

Currently, users need to visit professional hearing care centers to complete the hearing aid fitting process. The hearing aid fitting process, conducted with the assistance of certified audiologists and specialized equipment, is complex and time-consuming. Consequently, some self-fitting solutions have emerged for users. However, the effectiveness of current self-fitting solutions still falls short of achieving the precision level attainable through professional fine-tuning by audiologists.

### SUMMARY

The present disclosure provides a method for fitting a hearing aid and a hearing aid.

In a first aspect, the present disclosure provides a method for fitting a hearing aid, comprising: initializing signal-processing parameters of the hearing aid at different frequencies based on attribute data and hearing characteristic data of a user, wherein the signal-processing parameters comprising a gain; processing a hearing test signal to be played based on initialized values of the gain, and obtaining a processed hearing test signal, wherein the processed hearing test signal is configured for testing a hearing level of the user wearing the hearing aid; playing the processed hearing test signal and determining a hearing test result of the user using the hearing aid; in a case where the hearing test result indicates that the hearing aid does not meet a preset first hear-aiding requirement, adjusting the values of the gain of the hearing aid at different frequencies based on the hearing test result, and continuously iterating a hearing test based on the adjusted values of the gain until the hearing aid meets the preset first hear-aiding requirement.

In a second aspect, the present disclosure provides a hearing aid, comprising: a Bluetooth module; a speaker; and a processor, connected to both the Bluetooth module and the speaker. The Bluetooth module is configured for receiving hearing test signals at different frequencies and transmitting the received hearing test signals to the processor. The processor is configured for executing the operations of the method for fitting a hearing aid to adjust the values of the gain of the hearing aid at different frequencies, processing the hearing test signals based on the adjusted values of the gain, and transmitting the processed hearing test signals to the speaker. The speaker is configured for playing the processed hearing test signal.

### BRIEF DESCRIPTION OF THE DRAWINGS

In order to more clearly illustrate the technical solutions of the embodiments of the present disclosure, the drawings for the description of the embodiment will be described in brief. Obviously, the drawings in the following description are only some of the embodiments of the present disclosure. For a person of ordinary skill in the art, other drawings may be obtained based on the following drawings without any creative work.
FIG. 1 is a diagram illustrating the application environment of a method for fitting a hearing aid in one embodiment.
FIG. 2 is a flow chart of a method for fitting a hearing aid in one embodiment.
FIG. 3 is a flow chart of a method for fitting a hearing aid in another embodiment.
FIG. 4 is a schematic diagram showing initial values of the gain and values of the maximum output power at different frequencies obtained in one embodiment.
FIG. 5 is a flow chart of a method for fitting a hearing aid in yet another embodiment.
FIG. 6 is a detailed flow chart of a method for fitting a hearing aid in one embodiment.
FIG. 7 is a schematic diagram of a speech audiogram in one embodiment.
FIG. 8 is a detailed flow chart of a method for fitting a hearing aid in another embodiment.
FIG. 9 is a structure schematic view of an apparatus for fitting a hearing aid in one embodiment.
FIG. 10 is a structure schematic view of an apparatus for fitting a hearing aid in another embodiment.
FIG. 11 is an internal structural diagram of a computer device in one embodiment.

### DETAILED DESCRIPTION

To provide a clearer explanation of the objectives, technical solutions, and advantages of the present disclosure, further detailed description is provided below in conjunction with the accompanying drawings and embodiments. It should be understood that the specific embodiments described herein are intended solely to illustrate the application and do not limit its scope.

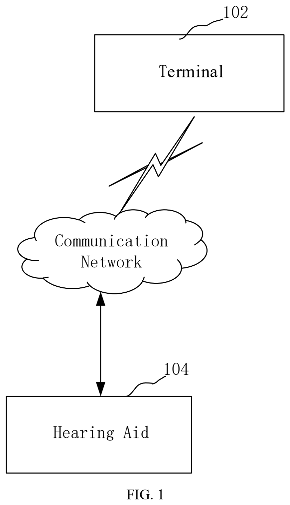

In some embodiments of the present disclosure, the method for fitting a hearing aid may be applied in an application environment as illustrated in FIG. 1. A terminal 102 wirelessly may communicate with a hearing aid 104 through other communication methods such as Bluetooth or wireless local area network.

In some embodiments, the terminal 102 may be installed with a hearing aid self-fitting application. A user may log in to the application to initiate the fitting operation. First, the terminal 102 may receive attribute data of the user inputting such as gender, age, and hearing characteristic data. The terminal 102 may transmit the attribute data and the hearing characteristic data of the user to the hearing aid 104. The hearing aid 104 may determine signal-processing parameters including a gain at different frequencies. Subsequently, the hearing aid 104 may initialize the signal-processing parameters (including the gain) at different frequencies. Then, the hearing aid 104 may process a hearing test signal to be played based on initialized values of the gain. Subsequently, a processed hearing test signal (such as a pure tone signal or a warble tone signal at different frequencies) may be played to guide the user for a self-administered hearing test. A hearing level of the user at different frequency points may be evaluated to determine a hearing test result of the user wearing the hearing aid 104. In a case where the hearing test result indicates that the hearing aid 104 may not meet a preset first hear-aiding requirement, the values of the gain of the hearing aid 104 at different frequencies may be adjusted based on the hearing test result. Based on the adjusted values of the gain, the hearing test signal to be played may continue to be processed. The hearing test may be conducted iteratively until the hearing aid 104 may meet the preset first hear-aiding requirement, thereby determining the optimal values of the gain of the hearing aid 104 at different frequency points.

In some embodiments, the terminal 102 may include, but is not limited to, various personal computers, laptops, smartphones, tablet computers, IoT devices, and portable wearable devices. IoT devices may comprise smart speakers, smart TVs, smart air conditioners, smart vehicle-mounted devices, projection equipment, etc. The portable wearable devices may include smartwatches, smart wristbands, and head-mounted devices. The head-mounted devices may be virtual reality (VR) devices, augmented reality (AR) devices, smart glasses, etc.

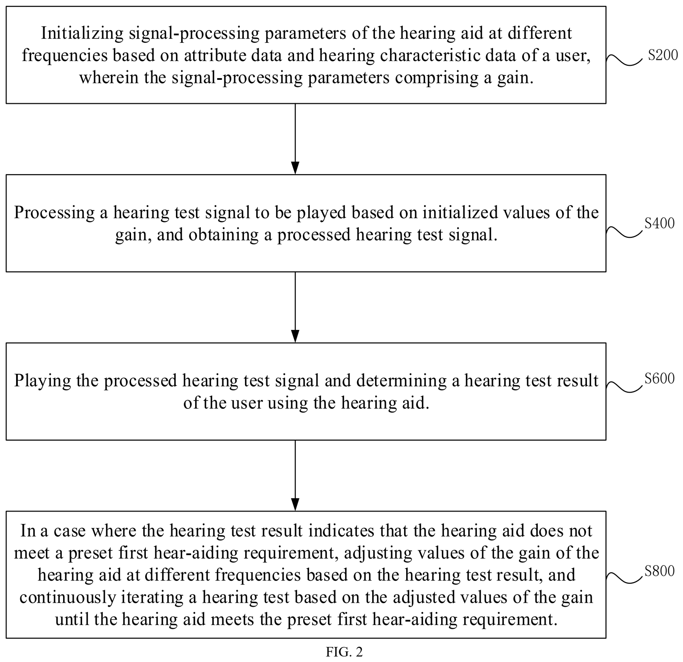

In some embodiments, as shown in FIG. 2, the present disclosure provides a method for fitting a hearing aid. The method may be implemented by taking the hearing aid 104 shown in FIG. 1 as an example for description. It may be understood that the method may also be applied to user terminals, servers, or systems comprising user terminals, servers, and hearing aids. In some embodiments, the method may include the following operations S200 to S600.

Operation S200: initializing signal-processing parameters of the hearing aid at different frequencies based on attribute data and hearing characteristic data of a user, wherein the signal-processing parameters comprising a gain.

The attribute data of the user may include, but is not limited to, age, gender, and disease conditions such as whether the user may suffer from any medical disorders. The hearing characteristic data may include, but is not limited to, wearing experience, uncomfortable loudness levels at different frequencies, and an audiogram in a preset time period, such as the past six months. The audiogram may comprise hearing data at 250Hz (Hertz)-500Hz-1kHz-2kHz-3kHz-4kHz-8kHz. In some embodiments, the attribute data of the user may be obtained using a questionnaire consultation. The attribute data of the user may also be obtained using an interactive system Q&A with the user. It may be understood that using the questionnaire consultation may help understand the degree and cause of hearing impairment of users, and assess whether the hearing impairment is due to medical conditions requiring treatment, such as microtia, otitis media, or sudden sensorineural hearing loss, to facilitate the subsequent determination of initial values of the gain.

The signal-processing parameters may refer to various parameters applied internally by the hearing aid to process audio signals, including but not limited to a gain, a maximum power output, a compression ratio, and a noise suppression. The gain may be configured for characterizing the degree of amplification applied by the hearing aid to sound signals at different frequencies. In some embodiments, initialized signal-processing parameters at different frequencies may include the values of the gain at 250Hz, 500Hz, 750Hz, 1kHz, 1.5kHz, 2kHz, 3kHz, 4kHz, 6kHz, and 8kHz.

In some embodiments, the hearing aid may be in communication with a user terminal, such as a smartphone. The user may input the basic attribute data, such as gender, age, and disease conditions, via the operation interface of the user terminal, and may provide hearing characteristic data, including the degree of hearing loss and the audiogram from the past six months. Subsequently, the user terminal may transmit the attribute data and the hearing characteristic data of the user to the hearing aid. The data processing module of the hearing aid may process the attribute data and the hearing characteristic data of the user using a standard gain calculation formula (such as NAL-NL2 (National Acoustic Laboratories - Non-Linear 2), DSL (Desired Sensation Level), etc.). The data processing module of the hearing aid may automatically calculate initial values of the gain of the hearing aid at different frequencies. In some embodiments, the initial values of the gain at G50 (soft sound)/G65 (medium sound)/G80 (loud sound) may be determined based on the attribute data and the hearing characteristic data of the user inputting. It may be understood that in a case where the user may have difficulty providing the audiogram from the past six months, in-situ audiometry may be performed on the user using the hearing aid to obtain the audiogram of the user. In some embodiments, in-situ audiometry may be conducted using the industry-standard Hughson-Westlake method, which is considered the gold standard in audiometry. During the in-situ audiometry process, the hearing aid may need to be calibrated to ensure the hearing aid may emit correct frequencies and accurate loudness levels. The calibration may guarantee measurement consistency and accuracy, thereby assisting audiologists and hearing care professionals in providing optimal hearing aid adjustment and fitting for the user.

In other embodiments, the user may also manually set the initial values of the gain of the hearing aid at different frequencies through the application interface. The hearing aid may initialize the values of the gain at different frequencies. Alternatively, the user terminal may process the attribute data and the hearing characteristic data of the user inputting according to a standard fitting formula to determine the initial values of the gain of the hearing aid at different frequencies. Subsequently, the determined initial values of the gain may be transmitted to the hearing aid. The hearing aid may initialize the values of the gain at different frequencies.

Operation S400: processing a hearing test signal to be played based on initialized values of the gain, and obtaining a processed hearing test signal, wherein the processed hearing test signal is configured for testing a hearing level of the user wearing the hearing aid.

The hearing test signal may typically include a series of standardized audio signals. The hearing test signal may be configured for evaluating the hearing thresholds and auditory comprehension abilities of the user wearing the hearing aid at different frequencies, thereby testing a hearing level of the user using the hearing aid. In some embodiments, the hearing test signal may include, but are not limited to, a pure-tone signal, a warble-tone signal, a speech test signal, and a noise signal. The pure-tone signal may refer to a single-frequency signal. The pure-tone signal may generally be configured for measuring the hearing thresholds of the user at different frequencies. The speech test signal may contain an actual speech sound, including monosyllabic words, disyllabic words, and others. The speech test signal may be configured for assessing the speech recognition ability of the user at different frequencies.

In some embodiments, after the hearing aid may have initialized the values of the gain, the hearing aid may process the hearing test signal to be played based on the initialized values of the gain to obtain a processed hearing test signal. For example, the initialized values of the gain of the hearing aid may be 20 dB. The hearing test signal may be a pure-tone signal with a frequency of 1 kHz and an initial intensity of 60 dB SPL (Sound Pressure Level). The pure-tone signal may be processed based on the initialized gain, whereby the initial intensity of 60 dB SPL may be increased to 80 dB SPL, obtaining a processed pure-tone signal with an intensity of 80 dB SPL.

Operation S600: playing the processed hearing test signal and determining a hearing test result of the user using the hearing aid.

In some embodiments, the hearing test result may include the hearing thresholds and speech recognition rates of the user at different frequencies.

In some embodiments, the user may select a relatively quiet location with low background noise to conduct the hearing test. The hearing aid may pre-store the hearing test signal to be played. After the hearing aid may initialize the values of the gain, the hearing test signal to be played may be processed based on the initialized values of the gain. Subsequently, the hearing test signal processed with the values of the gain may be played. The user may respond according to the heard hearing test signal, such as by clicking a button on the screen of the user terminal or providing verbal answers. The responses of the user to each type of the hearing test signal may be recorded. The hearing thresholds and the speech recognition rates of the user at different frequencies may be evaluated and determined to obtain the hearing test result of the user. In some embodiments, a sound-field test may be performed on the user using a pure-tone signal. The user may provide feedback on whether sounds are heard and its clarity. Based on feedback data of the user, the hearing test result may be obtained of the user wearing the hearing aid.

Operation S800: in a case where the hearing test result indicates that the hearing aid does not meet a preset first hear-aiding requirement, adjusting values of the gain of the hearing aid at different frequencies based on the hearing test result, and continuously iterating a hearing test based on the adjusted values of the gain until the hearing aid meets the preset first hear-aiding requirement.

In some embodiments, the first hear-aiding requirement may refer to an objective hear-aiding requirement, which may involve evaluating effectiveness of the hearing aid using scientific methods and technical means based on the hearing test result and relevant parameters of the hearing aid. For example, the first hear-aiding requirement may include, but is not limited to, the speech recognition rates of the user at different frequencies exceeding a preset speech recognition (e.g., 85%), and/or the hearing thresholds of the user at different frequencies approaching a desired threshold (e.g., 30 dB (decibel)).

In some embodiments, the hearing test results from the first round of the hearing test based on the initialized values of the gain may indicate that the hearing aid does not meet the first hear-aiding requirement. Speech recognition rates or hearing thresholds of the user at certain frequencies may not meet the first hear-aiding requirement. The values of the gain of the hearing aid at different frequencies may continue to be adjusted. Based on the adjusted values of the gain, the same hearing test signal may be processed. The processed hearing test signal may be played again. The hearing test result of the user may be recorded. Whether the hearing aid meets the first hear-aiding requirement may be evaluated again. In a case where the first hear-aiding requirement may be not meet, the values of the gain of the hearing aid may be adjusted again. The hearing test may be performed on the user again based on the adjusted values of the gain. The hearing test may be continuously iteratively conducted until the hearing aid meets the first hear-aiding requirement. For example, in a case where a speech recognition rate of the user in the high-frequency range may be relatively low, a value of the gain in the high-frequency range may be increased. Subsequently, the same hearing test signal to be played may continue to be processed using an increased value of the gain. The processed hearing test signal may be played again, testing the hearing level of the user wearing the hearing aid. The hearing test may be iterated until the hearing aid meets the first hear-aiding requirement. To maintain overall auditory balance, the values of the gain of the hearing aid at other frequency points may be adaptively adjusted after modifying the values of the gain at a specific frequency point. In some embodiments, in a case where an increase in gain at 2000 Hz may result in the user perceiving sounds between 1000 Hz and 3000 Hz as unbalanced or excessively sharp, adjustments to frequencies between 1000 Hz and 3000 Hz may also be performed to restore natural auditory balance.

The method for fitting a hearing aid described above, which may differ from approaches involving professional audiologists, may provide a solution that supports the user self-fitting of the hearing aid. Based on the attribute data and the hearing characteristic data of the user, the signal-processing parameters of the hearing aid at different frequencies may be initialized. The processed hearing test signal based on the initialized values of the gain may be played, testing the hearing level of the user wearing the hearing aid. The hearing test result of the user wearing the hearing aid may be obtained. The values of the gain of the hearing aid at different frequencies may be iteratively adjusted based on the hearing test result. After each adjustment of the values of the gain, the hearing level of the user with the hearing aid may be tested again. The optimal values of the gain settings most suitable for the user may be identified, enabling the hearing aid to meet the preset hear-aiding requirement. The aforementioned solution may combine automated hearing testing with a user feedback mechanism. By conducting repeated hearing test on the user and iteratively adjusting the values of the gain of the hearing aid at different frequencies based on the hearing test result, the users may quickly and accurately determine the optimal values of the gain for the hearing aid at each frequency point according to their own hearing experience and usage habits, thereby achieving better hearing aid performance. Furthermore, the entire process may be completed without requiring users to visit a professional fitting studio, as hearing aid fitting may also be performed at home, which may be more convenient.

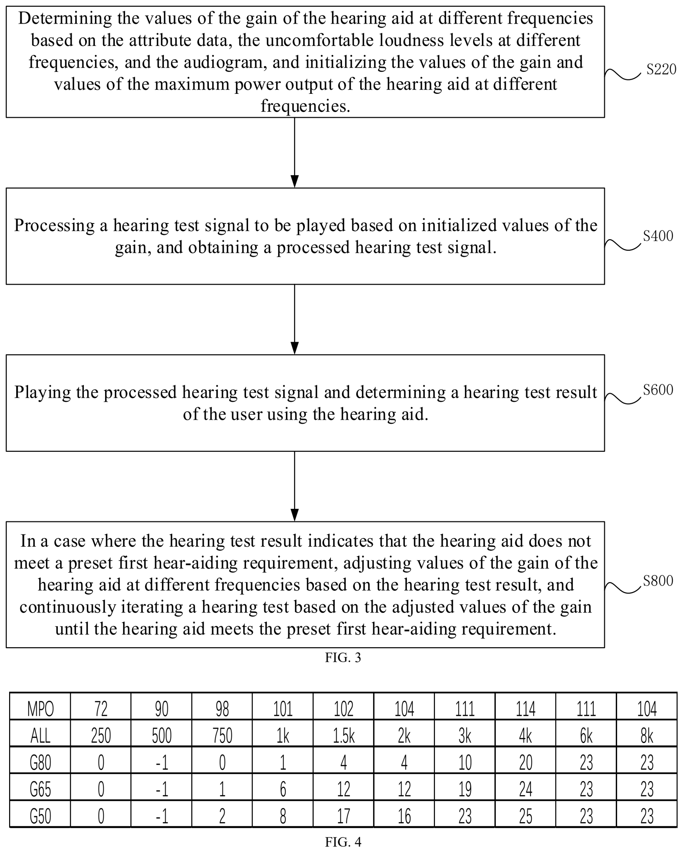

In some embodiments, during the initialization phase of the hearing aid, the signal-processing parameters that may need to be initialized may include a gain. The signal-processing parameters that may need to be initialized may also include a maximum power output. As shown in FIG. 3, in some embodiments, operation S200 may include the following operation.

Operation S220: determining the values of the gain of the hearing aid at different frequencies based on the attribute data, the uncomfortable loudness levels at different frequencies, and the audiogram, and initializing the values of the gain and values of the maximum power output of the hearing aid at different frequencies.

Uncomfortable loudness level (UCL) may refer to the minimum sound pressure level at which an individual begins to perceive sound as excessively loud or uncomfortable at a specific frequency. The threshold of the uncomfortable loudness level may vary from person to person. Even among patients with the same degree of hearing loss may exhibit significant differences in their uncomfortable loudness levels. Therefore, it may be necessary to determine the uncomfortable loudness levels at different frequencies for each user. In some embodiments, the application on the user terminal may play gradually increasing sounds at different frequency points. The user may be guided to provide feedback on tolerability through methods such as button presses or gestures. In a case where the user indicates intolerability, a corresponding threshold may be recorded as an uncomfortable loudness level at that frequency point. The maximum power output (MPO) may refer to the maximum sound pressure level that the hearing aid may output under a specific input signal.

In some embodiments, after obtaining the attribute data of the user (such as age, gender, etc.) and the hearing characteristic data (including the uncomfortable loudness levels at different frequencies and the audiogram), the attribute data and the hearing characteristic data of the user may be applied as inputs to the standard fitting formula, such as the NAL-NL2 formula. Through the NAL-NL2 formula, a mapping relationship from inputs (the audiogram + the user age + the gender + the disease conditions) to outputs (the initial gain) at different frequencies may be established. Subsequently, based on the uncomfortable loudness levels of the user at different frequencies, the values of the maximum power output at different frequencies may be determined. For example, the initial values of the gain of the hearing aid at different frequencies obtained through the NAL-NL2 formula may be illustrated in FIG. 4. The user terminal may transmit the initial values of the gain and the values of the maximum power output to the hearing aid. The hearing aid may initialize the initial values of the gain and the values of the maximum power output at different frequencies based on the initial values of the gain across different frequencies.

In this embodiment, based on the attribute data, the uncomfortable loudness levels at different frequencies, and the audiogram, combined with clinically summarized fitting prescription formulas, more accurate initial gain suitable for the user may be obtained, enabling the hearing aid to achieve better hearing assistance effects

In some embodiments, the initializing the values of the maximum power output of the hearing aid at different frequencies may include: determining the values of the maximum power output at different frequencies based on the uncomfortable loudness levels at different frequencies, and initializing the values of the maximum power output at different frequencies.

In some embodiments, the actual output of the hearing aid may be affected by environmental noise, input sound pressure level, and the inherent characteristics of the hearing aid itself. Therefore, the values of the maximum power output of the hearing aid may need to be set based on the uncomfortable loudness levels of the user.

In some embodiments, the values of the gain calculated by the NAL-NL2 formula may ensure that the output at different frequencies does not exceed the uncomfortable loudness levels of the user. Therefore, initializing the values of the maximum power output may involve setting the values of the maximum power output within the uncomfortable loudness levels of the user that does not surpass the uncomfortable loudness levels of the user. In some embodiment, the values of the maximum power output at different frequencies may be configured according to the principle that the maximum power output may approach but does not exceed the uncomfortable loudness levels at each frequency point, aiming to ensure maximum audibility without causing hearing damage to the user. In some embodiments, in a case where an uncomfortable loudness level of the user at 750 Hz is 100 dB, a value of the maximum power output at 750 Hz may be determined as 98 dB. It may be understood that, in practical applications, a fixed offset may also be set. The value of the maximum power output at corresponding frequencies may be determined based on the uncomfortable loudness levels at different frequencies. For example, with a fixed offset of 5 dB, in a case where the uncomfortable loudness level of the user at 750 Hz is 100 dB, the value of the maximum power output at 750 Hz may be determined as 95 dB.

In some embodiment, the values of the maximum power output at different frequencies may be determined based on the uncomfortable loudness levels at corresponding frequencies. The method may help enhance user comfort and may achieve personalized hearing aid configuration.

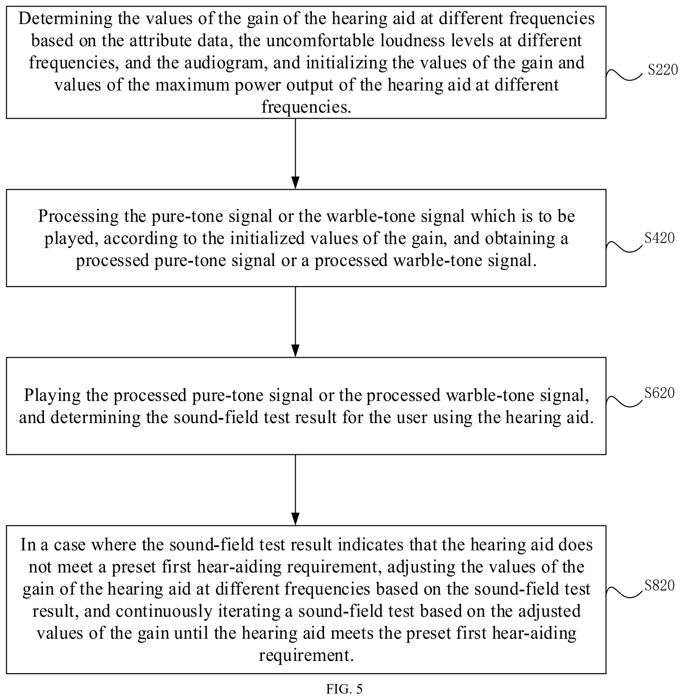

In some embodiments, as shown in FIG. 5, Operation S400 may include the following operation.

Operation S420: processing the pure-tone signal or the warble-tone signal which is to be played, according to the initialized values of the gain, and obtaining a processed pure-tone signal or a processed warble-tone signal.

Operation S600, including: operation 620: playing the processed pure-tone signal or the processed warble-tone signal, and determining the sound-field test result for the user using the hearing aid.

Operation S800, including: operation S820: in a case where the sound-field test result indicates that the hearing aid does not meet a preset first hear-aiding requirement, adjusting the values of the gain of the hearing aid at different frequencies based on the sound-field test result, and continuously iterating a sound-field test based on the adjusted values of the gain until the hearing aid meets the preset first hear-aiding requirement.

In some embodiments, the hearing test signal may include a pure-tone signal or a warble-tone signal. The pure-tone signal or the warble-tone signal may be configured for performing the sound-field test on the user. The hearing test result may include a sound-field test result. The warble-tone signal may be a type of sound signal whose frequency may vary over time. Unlike the pure-tone signal, the frequency of the warble-tone signal may not be fixed but may fluctuate within a certain range. The frequency variation of the warble-tone signal may make the signals closer to the sound variations in natural language, thereby providing an auditory experience more aligned with real-life situations. The sound-field test may be a method of evaluating hearing of the user by using speakers or similar sound source equipment in a controlled acoustic environment. The sound-field test result may include data such as hear-aiding thresholds of the user (minimum audible thresholds) at different frequencies.

In some embodiments, the hearing test (the sound-field test) on the user may be performed using the pure-tone signal or the warble-tone signal. After initializing the values of the gain of the hearing aid, the user terminal may generate a warble-tone signal within a predetermined frequency range. Alternatively, the user terminal may generate a pure-tone signal at a single frequency. The hearing aid may process the pure-tone signal or the warble-tone signal based on the initialized values of the gain. The pure-tone signal or the warble-tone signal intensity may be adjusted to enable the user to hear sounds at an appropriate level. Subsequently, the processed pure-tone signal or the processed warble-tone signal may be played.

In some embodiments, taking the playback of the warble-tone signal as an example, the hearing test may begin at 1kHz with a 40 dB sound signal. A frequency of the sound signal may gradually be increased to 2k, 4k, and 8kHz, followed by retesting the hearing threshold at 1kHz. The user may be guided to report whether the signal is audible through button presses, gestures, voice commands, or other means. For children, feedback may be obtained through games or other interactive methods. The hearing responses of the user at each frequency may be recorded to evaluate the hearing thresholds of the user. Furthermore, the subjective perceptions of the user may be inquired to understand their experience regarding sound volume, clarity, and other aspects, thereby obtaining more accurate sound-field test result. The hearing threshold initially measured at 1kHz may be recorded as X1, and the hearing threshold obtained from retesting at 1kHz may be recorded as X5. In a case where the absolute difference between X1 and X5 does not exceed 10 dB, the previously measured results may be considered reliable; otherwise, retesting may be required.

In some embodiments, taking the playback of the pure-tone signal as an example, the frequency points to be tested may first be determined. The hearing test may be conducted sequentially from low frequencies to high frequencies. For example, starting from a relatively low intensity level, the frequency may gradually be increased until the user may hear the sound signal. The user may be guided to report whether the signal is audible through button presses, gestures, voice commands, or other means. The hear-aiding thresholds of the user may be recorded at each frequency point, and an audiogram may be plotted based on the hear-aiding thresholds at different frequencies to obtain a hearing threshold curve.

After obtaining the hear-aiding thresholds of the user at different frequencies using the pure-tone signal or the warble-tone signal, in a case where it is determined that values of the gain at certain frequencies may be insufficient or excessive, the values of the gain of the hearing aid may be adjusted based on the sound-field test result. Subsequently, the sound-field test may be performed again based on the adjusted values of the gain until the gain setting at all key frequency points meet the hearing needs of the user, and the hearing aid may meet the preset first hear-aiding requirement.

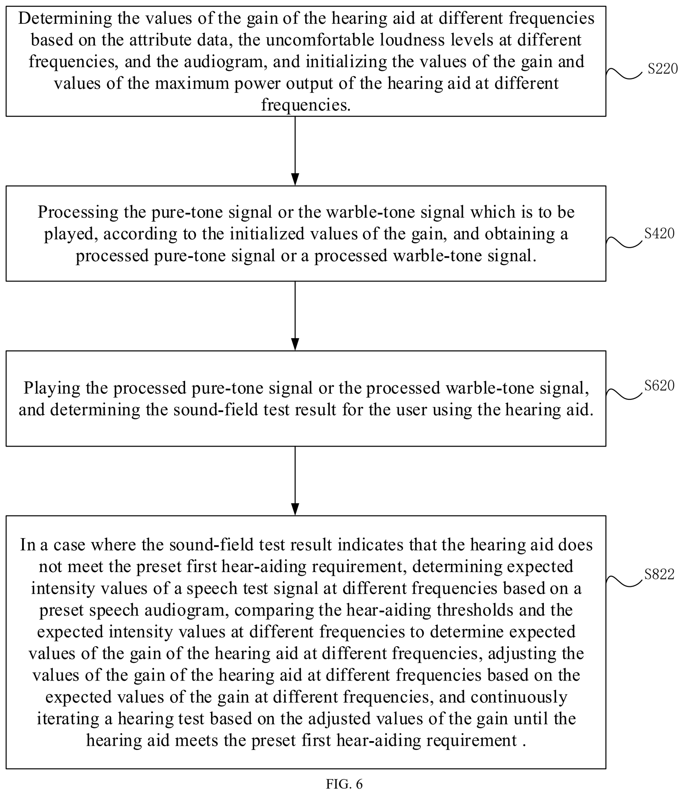

As shown in FIG. 6, in some embodiments, operation S820, including: operation S822: in a case where the sound-field test result indicates that the hearing aid does not meet the preset first hear-aiding requirement, determining expected intensity values of a speech test signal at different frequencies based on a preset speech audiogram, comparing the hear-aiding thresholds and the expected intensity values at different frequencies to determine expected values of the gain of the hearing aid at different frequencies, adjusting the values of the gain of the hearing aid at different frequencies based on the expected values of the gain at different frequencies, and continuously iterating a hearing test based on the adjusted values of the gain until the hearing aid meets the preset first hear-aiding requirement.

The hear-aiding thresholds, also referred to as the hearing thresholds, may indicate the minimum sound intensity level at which the user may detect and recognize a sound. On an audiogram, the hear-aiding thresholds may be expressed in decibels (dB HL, Hearing Level). For individuals with normal hearing, the hearing thresholds may typically range between 0 and 20dB HL. For those with hearing loss, the hearing thresholds without hearing assistance may be significantly higher than this range.

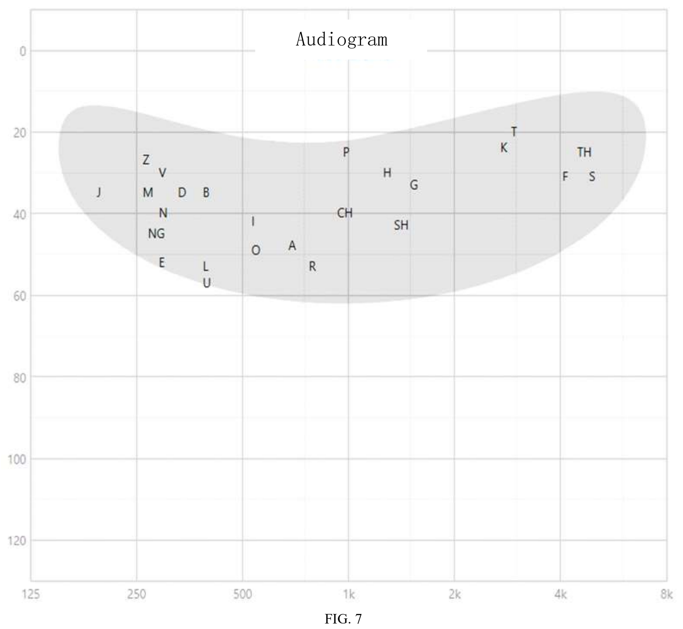

The speech audiogram, also known as a speech banana diagram, may be derived by measuring the frequency and intensity distribution of speech produced by a group of people speaking at normal volume using a sound level meter (refer to FIG. 7). The speech banana diagram may represent the frequency and intensity distribution range of speech in individuals with normal hearing. The speech banana diagram may serve as a key indicator for assessing whether an auditory communication of a person in daily life is effective. Since a sound intensity level of 20-40dB SPL may be comfortable for most users and may enable the most users to hear and understand speech as much as possible in normal conversation environments, the range of expected intensity values in this embodiment may be set between 20dB and 40dB.

In some embodiments, after obtaining the sound-field test result, the speech banana diagram may be configured for evaluating the effectiveness of the hearing aid. In some embodiments, in a case where all frequency points of the amplified hearing range fall within the speech banana diagram, it may indicate that the hearing aid provides excellent auditory compensation, suggesting that the user may be able to perceive speech sounds similarly to individuals with normal hearing. In some embodiments, the gain settings of the hearing aid may be configured such that the hearing thresholds of the user (the minimum volume level at which sounds become audible) may approach 20 dB HL as closely as possible across all frequencies covered by the speech banana diagram.

In some embodiments, after measuring the hear-aiding thresholds of the user at different frequencies, a comparison may be made to determine whether the hear-aiding thresholds are close to 20dB HL. In a case where the hear-aiding thresholds are not close to 20dB HL, an intensity difference between the hear-aiding thresholds and the expected intensity values at different frequencies may be determined. Based on the intensity difference, the expected values of the gain of the hearing aid at different frequencies may be calculated. Subsequently, the values of the gain of the hearing aid at different frequencies may be adjusted according to the expected values of the gain at corresponding frequencies. In some embodiments, in a case where a hear-aiding threshold of the user at 500 Hz is 45 dB HL, an intensity difference from an expected intensity value of 20 dB HL may be 25dB. The current value of the gain of the hearing aid may be increased by 25 dB relative to the original value of the gain. It may be understood that adjustments to the values of the gain of the hearing aid are typically incremental, and multiple fine-tuning iterations may be required to identify the most suitable settings for the user.

In some embodiments, by incorporating the speech audiogram to determine the expected intensity, the adjustment range of the values of the gain of the hearing aid at different frequencies may be determined, thereby enabling the identification of optimal values of the gain at various frequencies to achieve the best hearing compensation outcome.

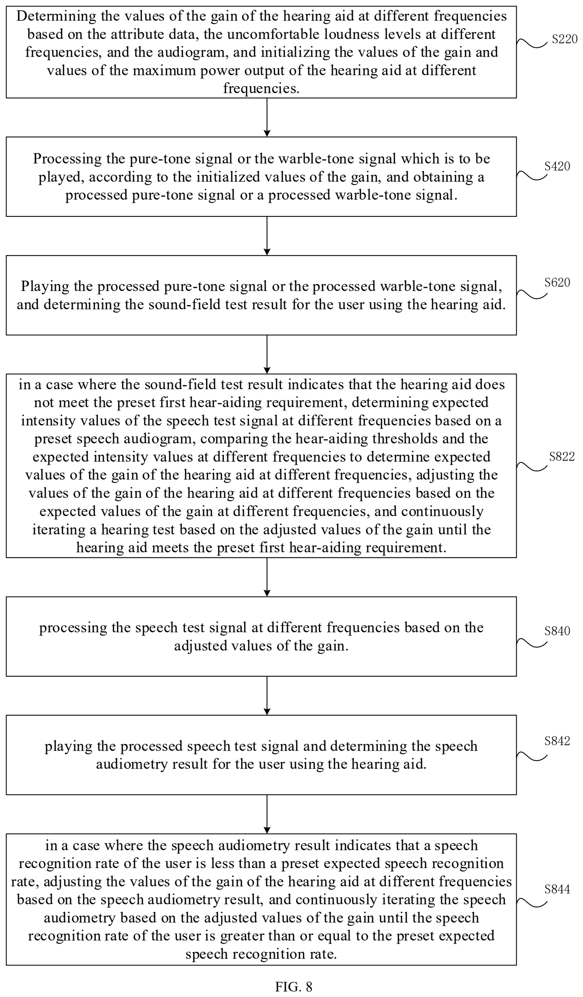

In some embodiments, in addition to testing the hearing threshold of the user, speech recognition thresholds of the user at different frequencies may also be tested. As shown in FIG. 8, in some embodiments, after operation S822, the method may further include the following operations.

Operation S840: processing the speech test signal at different frequencies based on the adjusted values of the gain.

Operation S842: playing the processed speech test signal and determining the speech audiometry result for the user using the hearing aid.

Operation S844: in a case where the speech audiometry result indicates that a speech recognition rate of the user is less than a preset expected speech recognition rate, adjusting the values of the gain of the hearing aid at different frequencies based on the speech audiometry result, and continuously iterating the speech audiometry based on the adjusted values of the gain until the speech recognition rate of the user is greater than or equal to the preset expected speech recognition rate.

In some embodiments, taking the sound-field test and the speech audiometry for the user as an example. The hearing test result may include the sound-field test result and the speech audiometry result, while the hearing test signal may include the pure-tone signal or the warble-tone signal, as well as the speech test signal.

The speech recognition rate may refer to an ability of an individual to correctly identify speech under specific conditions. The speech recognition rate may typically be expressed as a percentage. For example, in a case where a person correctly identifies 70 out of 100 words, the speech recognition rate of the person may be 70%. In this embodiment, an expected speech recognition rate may be set at 85%. It may be understood that in other embodiments, the expected speech recognition rate may also be set at other values, such as 90% or 80%, without being specifically limited herein. The speech audiometry result may include data such as the speech recognition rate and the speech recognition thresholds of the user at different frequencies.

In some embodiments, the speech audiometry may be performed on the user by preparing 25 monosyllabic words or disyllabic words (i.e., the speech test signals) covering common speech frequency ranges. Subsequently, the hearing aid may operate based on the adjusted values of the gain to play the 25 monosyllabic words or disyllabic words. After each word is played, the user may be asked to repeat or select the correct word. The responses of the user may be recorded to determine whether the words are correct. The ratio of correctly answered words to the total number of words may be calculated, i.e., speech recognition rate = (number of correct answers / total number of words) × 100%. In a case where a speech recognition rate at a certain frequency point measured in this round does not reach 85%, the speech audiometry results may be analyzed to identify frequency bands with lower recognition rates, and the values of the gain in the corresponding frequency bands may be adjusted. Subsequently, the 25 monosyllabic words or disyllabic words may be processed based on the adjusted values of the gain. Speech audiometry may continue to be performed on the user to verify whether the new gain settings effectively improve the speech recognition rate. The above process may be iterated, with speech audiometry repeated after each gain adjustment, until the speech recognition rate of the user wearing the hearing aid at each frequency point reaches or exceeds 85%.

In some embodiments, using continuous iterative adjustments and the hearing test, personalized gain optimization tailored to each specific needs of the user may be achieved. The hearing aid settings may be progressively refined until an optimal hearing state is reached, enabling the user to attain the highest level of speech comprehension.

Based on the speech audiometry result, the values of the gain of the hearing aid may be adjusted by analyzing the words that the user incorrectly identified. In some embodiments, based on the speech audiometry result, adjusting the values of the gain of the hearing aid at different frequencies, including: extracting misrecognized words that the user has answered incorrectly from the speech audiometry result, determining Ling's Six Sounds corresponding to the misrecognized words and a target frequency corresponding to the Ling's Six Sounds, and adjusting the values of the gain of the hearing aid at the target frequency.

The Ling's six tones (m, u, a, i, sh, s) encompass important frequency components in speech, wherein:
m: correspond to low-frequency components.
u: correspond to low-frequency components.
a: correspond to mid-frequency components.
i: correspond to mid-frequency components.
sh: correspond to high-frequency components.
s: correspond to high-frequency components.

Building on the previous embodiment, during each round of speech audiometry, misrecognized words and correctly identified words by the user may be recorded. In a case where the speech recognition rate of the user may fall below the expected speech recognition rate of 85%, the values of the gain of the hearing aid at relevant frequency points may be adjusted based on the Ling's Six Sounds corresponding to the misrecognized words. For example, in a case where the user may exhibit difficulty recognizing high-frequency words, the value of the gain in the high-frequency range (e.g., 3000 Hz and 3500 Hz) may be increased. Subsequently, the speech audiometry may be repeated using the adjusted values of the gain to evaluate the effectiveness of the modifications. In a case where the recognition rate still may fail to meet the expected speech recognition rate (≥85%), further analysis of misrecognized words and adjustments to the hearing aid settings may be iteratively performed until the speech recognition rate of the user may reach the desired level. Furthermore, in other embodiments, fine-tuning may also be conducted based on the specific feedback of the user to achieve an even higher recognition rate.

In some embodiments, assuming a first-round speech audiometry result of the user indicate 18 correctly identified words, yielding a speech recognition rate of 72%, analysis of the misrecognized words may reveal frequent errors in high-frequency phonemes (e.g., "sh" and "s"). Accordingly, the values of the gain of the hearing aid at frequency points such as 3000 Hz and 3500 Hz may be adjusted. It may be understood that the values of the gain at other frequency points may also be adaptively modified to maintain spectral balance. Based on the adjusted values of the gain, a second round of speech audiometry may be conducted. In a case where the results of this round show 22 correctly identified words, corresponding to a speech recognition rate of 88%, which meets or exceeds the target rate of 85%, the speech audiometry may be concluded.

In some embodiments, by analyzing the Ling's Six Sounds corresponding to the misrecognized words of the user, the parameter settings of the hearing aid may be progressively optimized. This approach may enable the user to achieve a high speech recognition rate across different frequencies. Consequently, not only may the auditory experience of the user be improved, but their communication abilities in daily life may also be enhanced.

In some embodiments, after adjusting the values of the gain of the hearing aid at the target frequency, the method further including: in a case where the number of gain adjustments reaches an upper limit, the speech audiometry result indicates that the speech recognition rate of the user at the target frequency is less than the preset expected speech recognition rate, and the sound-field test result indicates that the user has heard the pure-tone signal or the warble-tone signal at the target frequency, playing a preset rehabilitation training sample to conduct rehabilitation training for the user.

In some embodiments, since the speech recognition rate of the user may be influenced by multiple factors, the maximum number of gain adjustments may be set to 5 to ensure the smooth progress of the test. In some embodiments, the rehabilitation training samples may include short audio recordings from different environmental scenarios.

Building on the previous embodiment, during the speech audiometry process, in a case where the number of gain adjustments reaches 5 and the speech recognition rate of the user at a specific target frequency still may fail to meet the expected speech recognition rate of 85%, but the speech banana diagram of the user indicates that the user may hear the pure-tone signal or the warble-tone signal at the target frequency, it may be determined that the user is capable of detecting sounds but unable to comprehend the meaning of the speech signals. In some embodiments, targeted rehabilitation training may be provided to train the brain of the user in speech comprehension. In some embodiments, the user terminal may display a corresponding rehabilitation training interface. The hearing aid may play short audio recordings from different environmental scenarios, guiding the user to listen and attempt to understand the dialogue content. The user may complete keyword filling exercises on the interface of the terminal to accomplish the hearing rehabilitation training. It may be understood that throughout the rehabilitation training process, the hearing aid may need to provide clear audio input and maintain stable auditory conditions across different training stages, thereby helping the user gradually improve speech comprehension skills. In other embodiments, alternative rehabilitation samples may also be utilized for user training, without being limited to the described approach.

In some embodiments, by integrating rehabilitation training into the self-fitting process of the hearing aid, the hearing aid may not only assist the user in detecting sounds but also facilitate the transition from mere sound detection to speech comprehension.

In addition to the aforementioned method for fitting a hearing aid based on objective data, subjective fitting requirements by the user may also be incorporated. In some embodiments, after the continuously iterating the hearing level of the user wearing the hearing aid based on the adjusted values of the gain, the method further including: acquiring hearing test data fed back by the user and adjusting the values of the gain of the hearing aid at different frequencies based on the hearing test data until the hearing aid meets a preset second hear-aiding requirement.

In some embodiments, the second hear-aiding requirement may refer to a subjective hear-aiding requirement. Specifically, the second hear-aiding requirement may indicate that during actual use of the hearing aid, the user may determine whether the hearing aid may achieve the expected hear-aiding effect based on personal experience and perception, and may assess the satisfaction level with the hearing aid. In a case where the satisfaction level may exceed a preset satisfaction threshold, such as 90%, the hearing aid may be determined to meet the second hear-aiding requirement. It may be understood that in other embodiments, the user may also be guided to rate the satisfaction of the user with the hearing aid. In a case where the satisfaction score may exceed a preset score threshold, such as 9 points, the hearing aid may be determined to meet the second hear-aiding requirement. The satisfaction threshold or satisfaction score may also be set to other values, which are not uniquely limited herein.

In some embodiments, after the hearing aid meets objective hear-aiding requirements through the sound-field test and the speech audiometry, the values of the gain of the hearing aid may be further adjusted based on the subjective auditory perception of the user, enabling the user to determine whether the hearing aid may meet expected hearing assistance outcomes of the user. In some embodiments, the user terminal may engage in a Q&A interaction with the user via the interface of the application. For example, the user may be inquired whether the user experiences self-talking sound, echo or abnormal sound of the user wearing the hearing aid, and whether they may hear any other abnormal sounds in daily life. The user may provide feedback on their actual hearing experience through voice responses, pressing buttons on the hearing aid, or gently tapping the hearing aid. The hearing aid may record the user feedback of the user as hearing test data. Subsequently, based on the hearing test data, the values of the gain of the hearing aid at different frequencies may be adjusted until the satisfaction of the user with the hearing aid exceeds the preset satisfaction threshold, thereby completing the self-fitting process of the hearing aid.

In some embodiments, after obtaining the user feedback of the user on the hearing test data, the values of the gain of the hearing aid at different frequencies may be adjusted based on the hearing test data and known hearing characteristics of the user (such as wearing experience and discomfort thresholds at different frequencies), until the satisfaction of the user with the hearing aid may exceed a preset satisfaction threshold, thus completing the self-fitting of the hearing aid.

In some embodiments, the values of the gain of the hearing aid may be finely adjusted based on wearing experience and an acceptance level of the user. For first-time users, after determining a target value of the gain, 80% of the target value of the gain may be provided in the first month, 90% in the second month, and 100% in subsequent months, allowing the user to adapt gradually. For example, in a case where during a self-fitting process, the user may report being unable to hear the sound of an air conditioner, the hearing aid may identify that the G50 (soft sound) gain is too low. Subsequently, the G50 value of the gain across all frequency points may be gradually increased by a preset step size (e.g., 2 dB) until the user achieves a comfortable auditory experience. In a case where the user may report that their own voice sounds too loud, the G80 (loud sound) value of the gain may be identified as too high. Accordingly, the G80 value of the gain across all frequency points may be gradually reduced by a preset step size (e.g., 2 dB) until the user reaches a comfortable auditory experience.

In some embodiments, the combination of objective and subjective adjustments may enable a more comprehensive evaluation. This approach may not only allow the hearing aid to achieve better auditory compensation effects but may also empower users to perform personalized fine-tuning based on individual needs and practical scenarios, thereby ensuring the hearing aid may better align with the user actual requirements of the user.

To provide a clearer explanation of the method for fitting a hearing aid described in the embodiments of the present disclosure, the following detailed description may be made in conjunction with an embodiment. The embodiment may include the following operations.

Operation S100: determining the values of the gain of the hearing aid at different frequencies based on the attribute data of the user, the uncomfortable loudness levels at different frequencies, and the audiogram.

Operation S102: determining the values of the maximum power output at different frequencies based on the uncomfortable loudness levels at different frequencies.

Operation S104: initializing the values of the gain and the values of the maximum power output of the hearing aid at different frequencies.

Operation S106: processing the pure-tone signal or the warble-tone signal which is to be played, according to the initialized gain, and obtaining a processed pure-tone signal or a processed warble-tone signal, playing the processed pure-tone signal or the processed warble-tone signal to perform sound-filed test on the user, and determining the sound-field test result for the user using the hearing aid.

Operation S108: determining the expected intensity values of a speech test signal at different frequencies based on a preset speech spectrogram in a case where the sound-field test result indicates that the hearing aid does not meet the preset objective hear-aiding requirement, and comparing the hear-aiding thresholds and the expected intensity values at different frequencies to determine expected values of the gain of the hearing aid at different frequencies.

Operation S110: adjusting the values of the gain of the hearing aid at different frequencies based on the expected values of the gain at different frequencies, and continuously iterating the sound-field test based on the adjusted values of the gain until the hearing thresholds of the user at different frequencies approach the expected thresholds.

Operation S112: processing the speech test signal at different frequencies based on the adjusted values of the gain, and obtaining a processed speech test signal, playing the processed speech test signal and determining the speech audiometry result for the user using the hearing aid.

Operation S114: in a case where the speech audiometry result indicates that a speech recognition rate of the user is less than a preset expected speech recognition rate, determining Ling's Six Sounds corresponding to the misrecognized words and a target frequency corresponding to the Ling's Six Sounds, adjusting the value of the gain of the hearing aid at the target frequency, and continuously iterating the speech audiometry based on the adjusted values of the gain until the speech recognition rate of the user is greater than or equal to the preset expected speech recognition rate

In some embodiments, during the speech audiometry process, in a case where the number of gain adjustments reaches 5 and the speech recognition rate of the user at a specific target frequency still does not meet the expected speech recognition rate of 85%, but the speech banana diagram of the user indicates that the user may hear the pure-tone signal or the warble-tone signal at the target frequency, it may be determined that targeted rehabilitation training is required for the user. In some embodiments, the user terminal may display a corresponding rehabilitation training interface. The hearing aid may play short audio recordings from different environmental scenarios, guiding the user to listen and attempt to understand the dialogue content. The user may then complete keyword filling exercises on the interface of the terminal to accomplish the hearing rehabilitation training.

Operation S116: acquiring hearing test data fed back by the user, adjusting the values of the gain of the hearing aid at different frequencies based on the hearing test data until the hearing aid meets the preset subjective hear-aiding requirement.

It may be understood that although the operations in the flowcharts related to the aforementioned embodiments are shown sequentially as indicated by arrows, these operations may not necessarily be executed in the order indicated by the arrows. Unless explicitly stated herein, there may be no strict sequential limitation on the execution of these operations, and they may be executed in other orders. Moreover, at least a portion of the operations in the flowcharts related to the aforementioned embodiments may include multiple operations or stages, which may not necessarily be completed at the same time but may be executed at different times. The execution order of these operations or stages may not necessarily be sequential and may alternate or be performed interchangeably with other operations or at least a portion of the operations or stages of other operations.

Based on the same inventive concept, embodiments of the present disclosure further provide an apparatus for fitting a hearing aid for implementing the above-mentioned method for fitting a hearing aid. The problem-solving solutions provided by the apparatus are similar to those described in the aforementioned method. Therefore, specific limitations in one or more embodiments of the hearing aid fitting apparatus provided below may be referred to the limitations described for the method for fitting a hearing aid above, and will not be repeated here.

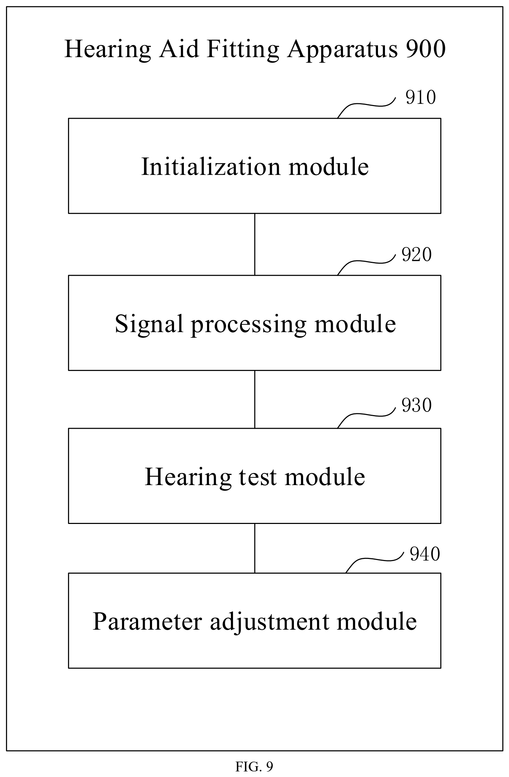

In some embodiments, as shown in FIG. 9, the present disclosure provides an apparatus for fitting hearing aid 900, which may comprise: an initialization module 910, a signal processing module 920, a hearing test module 930, and a parameter adjustment module 940, wherein:

The initialization module 910 may be configured for initializing signal-processing parameters of the hearing aid at different frequencies based on attribute data and hearing characteristic data of a user, wherein the signal-processing parameters comprising a gain.

The signal processing module 920 may be configured for processing a hearing test signal to be played based on initialized values of the gain, and obtaining a processed hearing test signal, wherein the processed hearing test signal is configured for testing a hearing level of the user wearing the hearing aid.

The hearing test module 930 may be configured for playing the processed hearing test signal and determining a hearing test result of the user using the hearing aid.

The parameter adjustment module 940 may be configured for, in a case where the hearing test result indicates that the hearing aid does not meet a preset first hear-aiding requirement, adjusting the values of the gain of the hearing aid at different frequencies based on the hearing test result, and continuously iterating a hearing test based on the adjusted values of the gain until the hearing aid meets the preset first hear-aiding requirement.

In some embodiments, the hearing test module 930 may further be configured for processing the pure-tone signal or the warble-tone signal which is to be played, according to the initialized values of the gain, and obtaining a processed pure-tone signal or a processed warble-tone signal, and playing the processed pure-tone signal or the processed warble-tone signal, and determining the sound-field test result for the user using the hearing aid.

In some embodiments, the parameter adjustment module 940 may further be configured for determining expected intensity values of speech test signals at different frequencies based on a preset speech audiogram, comparing the hear-aiding thresholds and the expected intensity values at different frequencies to determine expected values of the gain of the hearing aid at different frequencies, and adjusting the values of the gain of the hearing aid at different frequencies based on the expected values of the gain at different frequencies.

In some embodiments, the hearing test module 930 may further be configured for processing a speech test signal at different frequencies based on the adjusted values of the gain, and obtaining a processed speech test signal, wherein the speech test signal is configured for performing a speech audiometry for the user, playing the processed speech test signal and determining the speech audiometry result for the user using the hearing aid, and in a case where the speech audiometry result indicates that a speech recognition rate of the user is less than a preset expected speech recognition rate, adjusting the values of the gain of the hearing aid at different frequencies based on the speech audiometry result, and continuously iterating the speech audiometry based on the adjusted values of the gain until the speech recognition rate of the user is greater than or equal to the preset expected speech recognition rate.

In some embodiments, the parameter adjustment module 940 may further be configured for extracting misrecognized words that the user has answered incorrectly from the speech audiometry result, determining Ling's Six Sounds corresponding to the misrecognized words and a target frequency corresponding to the Ling's Six Sounds, and adjusting a value of the gain of the hearing aid at the target frequency.

In some embodiments, the hearing characteristic data may include uncomfortable loudness levels at different frequencies and an audiogram in a preset time period. The initialization module 910 may further be configured for determining the values of the gain of the hearing aid at different frequencies based on the attribute data, the uncomfortable loudness levels at different frequencies, and the audiogram, and initializing the values of the gain and the values of the maximum power output of the hearing aid at different frequencies.

In some embodiments, the initialization module 910 may further be configured for determining the values of the maximum power output at different frequencies based on the uncomfortable loudness levels at different frequencies, and initializing the values of the maximum power output at different frequencies.

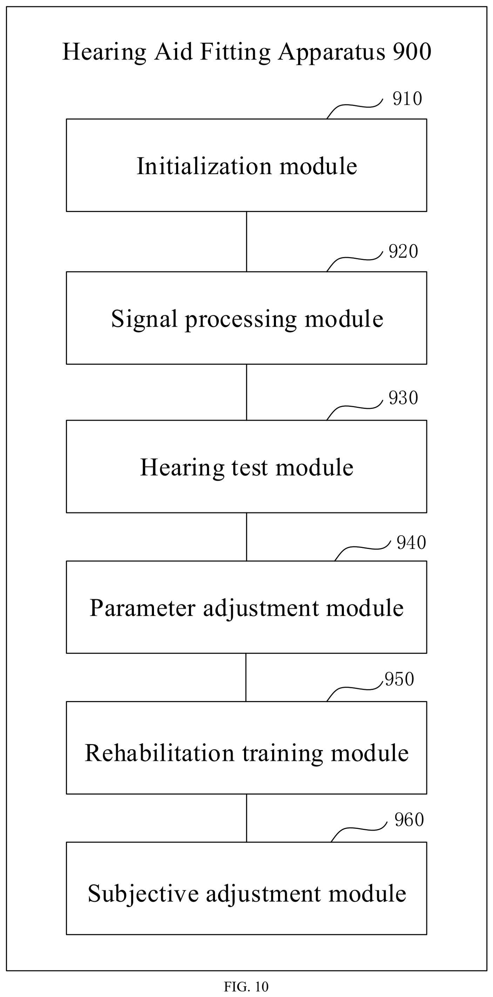

As shown in FIG. 10, in some embodiments, the apparatus may further comprise a rehabilitation training module 950. The rehabilitation training module 950 may be configured for, in a case where the number of gain adjustments reaches an upper limit, the speech audiometry result indicates that the speech recognition rate of the user at the target frequency is less than the preset expected speech recognition rate, and the sound-field test result indicates that the user has heard the pure-tone signal or the warble-tone signal at the target frequency, playing a preset rehabilitation training sample to conduct rehabilitation training for the user.

As shown in FIG. 10, in some embodiments, the apparatus may further comprise a subjective adjustment module 960, configured for acquiring hearing test data fed back by the user, and adjusting the values of the gain of the hearing aid at different frequencies based on the hearing test data until the hearing aid meets a preset second hear-aiding requirement.

In some embodiments, the subjective adjustment module 960 may further be configured for acquiring hearing test data fed back by the user, and adjusting the values of the gain of the hearing aid at different frequencies based on the hearing test data until the hearing aid meets a preset second hear-aiding requirement.

The various modules in the aforementioned hearing aid fitting apparatus may be implemented fully or partially through software, hardware, or a combination thereof. These modules may be embedded in or independent of the processor of a computer device in the form of hardware, or may be stored in the memory of the computer device in the form of software, thereby enabling the processor to invoke and execute the operations corresponding to each module.

In some embodiments, the present disclosure provides a hearing aid, which may comprise a Bluetooth module, a processor, and a speaker, wherein the processor may be connected to the Bluetooth module and the speaker, and wherein:

The Bluetooth module may be configured for receiving a hearing test signal at different frequencies and transmitting the received hearing test signal to the processor. The processor may execute the aforementioned embodiments of the method for fitting a hearing aid to adjust the values of the gain of the hearing aid at different frequencies, process the hearing test signal based on the adjusted gain, and transmit the processed hearing test signal to the speaker. The speaker is configured for playing the processed hearing test signal.

In some embodiments, the hearing aid may establish a connection with an external Bluetooth testing device, such as a smartphone, via the Bluetooth module. The hearing aid may receive hearing test signal transmitted from the smartphone through the Bluetooth module. The received hearing test signal may then be forwarded to the processor. The processor may process the hearing test signal and adjust the values of the gain of the hearing aid at different frequencies until the hearing aid meets the preset hear-aiding requirement.

It may be understood that the components listed above for the hearing aid may only represent those relevant to the solution of the present disclosure and may not constitute a limitation on the hearing aid to which the solution of the present disclosure is applied. In addition to the components listed above, the hearing aid may also include a power module, a microphone, and other components.

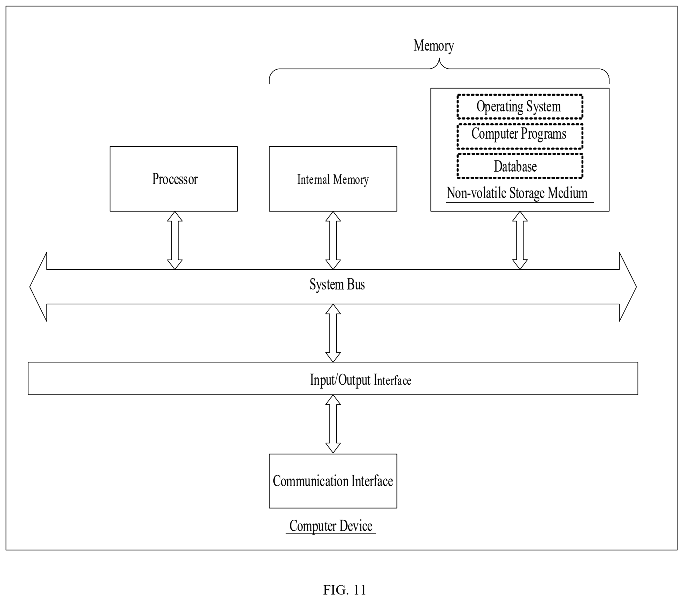

In some embodiments, a computer device may be provided. The computer device may be a server, and its internal structure diagram may be shown in FIG. 11. The computer device may comprise a processor, a memory, an input/output interface (I/O), and a communication interface. The processor, memory, and input/output interface may be connected via a system bus, and the communication interface may be connected to the system bus through the input/output interface. The processor of the computer device may be configured to provide computing and control capabilities. The memory of the computer device may include a non-volatile storage medium and an internal memory. The non-volatile storage medium may store an operating system, computer programs, and a database. The internal memory may provide an environment for the operation of the operating system and computer programs in the non-volatile storage medium. The database of the computer device may be configured to store user attribute data, hearing characteristics, hearing test data, and other relevant information. The input/output interface of the computer device may be configured to facilitate information exchange between the processor and external devices. The communication interface of the computer device may be configured to establish network communication with external terminals. The computer programs, when executed by the processor, may implement a method for fitting a hearing aid.

Those skilled in the art may understand that the structure shown in FIG. 11 may only be a block diagram of a partial structure related to the solution of the present application and may not constitute a limitation on the computer device to which the solution of the present disclosure is applied. A specific computer device may include more or fewer components than those shown in the figure, or may combine certain components, or may have a different arrangement of components.

In some embodiments, a computer device may be provided, comprising a memory and a processor. The memory may store a computer program, and the processor, when executing the computer program, may implement the operations in any of the aforementioned embodiments of the method for fitting a hearing aid.

In some embodiments, a computer-readable storage medium may be provided, having stored there on a computer program that, when executed by a processor, causes the processor to implement the operations in any of the aforementioned method embodiments of the method for fitting a hearing aid.

In some embodiments, a computer program product may be provided, comprising a computer program that, when executed by a processor, causes the processor to implement the steps in any of the aforementioned embodiments of the method for fitting a hearing aid.

It should be noted that the user information (including but not limited to user device information, personal user information, etc.) and data (including but not limited to data used for analysis, stored data, displayed data, etc.) involved in this application are all authorized by users or fully authorized by relevant parties. Furthermore, the collection, use, and processing of related data must comply with relevant regulations.

Those skilled in the art may understand that all or part of the processes in the methods of the above embodiments may be implemented by a computer program instructing relevant hardware. The computer program may be stored in a non-volatile computer-readable storage medium. When the computer program is executed, it may include the processes of the embodiments of the above method. Any references to memory, databases, or other media in the embodiments provided in the present disclosure may include at least one of non-volatile memory and volatile memory.

Non-volatile memory may include read-only memory (ROM), magnetic tape, floppy disks, flash memory, optical storage, high-density embedded non-volatile memory, resistive random-access memory (ReRAM), magneto resistive random-access memory (MRAM), ferroelectric random-access memory (FRAM), phase-change memory (PCM), graphene memory, and others. Volatile memory may include random-access memory (RAM) or external cache memory, among others. By way of illustration and not limitation, RAM may take various forms, such as static random-access memory (SRAM) or dynamic random-access memory (DRAM), among others.

The databases involved in the embodiments provided in the present disclosure may include at least one of relational databases and non-relational databases. Non-relational databases may include blockchain-based distributed databases, among others, without being limited thereto.

The processors involved in the embodiments provided in the present disclosure may include general-purpose processors, central processing units (CPUs), graphics processing units (GPUs), digital signal processors (DSPs), programmable logic devices, quantum computing-based data processing logic devices, artificial intelligence (AI) processors, and others, without being limited thereto.

The technical features of the above embodiments may be arbitrarily combined. To simplify the description, not all possible combinations of the technical features in the above embodiments have been described. However, as long as there is no contradiction in the combination of these technical features, such combinations shall be considered to fall within the scope documented in the present disclosure.

The above-described embodiments merely represent several implementations of the present disclosure and are described in specific and detailed terms, but should not be construed as limiting the patent scope of the present disclosure. It should be noted that those of ordinary skill in the art may make several modifications and improvements without departing from the concept of the present disclosure, all of which shall fall within the protection scope of the present disclosure. Therefore, the protection scope of the present disclosure shall be subject to the appended claims.

## Claims

1. A method for fitting a hearing aid, comprising:
initializing (200) signal-processing parameters of the hearing aid at different frequencies based on attribute data and hearing characteristic data of a user, wherein the signal-processing parameters comprising a gain;
processing (400) a hearing test signal to be played based on initialized values of the gain, and obtaining a processed hearing test signal, wherein the processed hearing test signal is configured for testing a hearing level of the user wearing the hearing aid;
playing (600) the processed hearing test signal and determining a hearing test result of the user using the hearing aid; and
in a case where the hearing test result indicates that the hearing aid does not meet a preset first hear-aiding requirement, adjusting (800) values of the gain of the hearing aid at different frequencies based on the hearing test result, and continuously iterating a hearing test based on the adjusted values of the gain until the hearing aid meets the preset first hear-aiding requirement.

2. The method as claimed in claim 1, wherein the hearing test signal comprises a pure-tone signal or a warble-tone signal, and the hearing test result comprises a sound-field test result;
the processing (400) a hearing test signal to be played based on the initialized values of the gain and obtaining a processed hearing test signal, comprises:
processing (420) the pure-tone signal or the warble-tone signal which is to be played, according to the initialized values of the gain, and obtaining a processed pure-tone signal or a processed warble-tone signal, wherein the pure-tone signal or the warble-tone signal is configured for performing a sound-field test for the user;
the playing (600) the processed hearing test signal and determining a hearing test result of the user using the hearing aid, comprises:
playing (620) the processed pure-tone signal or the processed warble-tone signal, and determining the sound-field test result for the user using the hearing aid.

3. The method as claimed in claim 2, wherein the sound-field test result comprises hear-aiding thresholds at different frequencies for the user using the hearing aid;
adjusting the values of the gain of the hearing aid at different frequencies based on the sound-field test result, comprises:
determining expected intensity values of a speech test signal at different frequencies based on a preset speech audiogram;
comparing the hear-aiding thresholds and the expected intensity values at different frequencies to determine expected values of the gain of the hearing aid at different frequencies; and
adjusting the values of the gain of the hearing aid at different frequencies based on the expected values of the gain at different frequencies.

4. The method as claimed in claim 2, wherein the hearing test signal further comprises the speech test signal at different frequencies, and the hearing test result further comprises a speech audiometry result;
after adjusting the values of the gain of the hearing aid at different frequencies based on the sound-field test result until the hearing aid meets the preset first hear-aiding requirement, the method further comprises:
processing (840) the speech test signal at different frequencies based on the adjusted values of the gain, and obtaining a processed speech test signal, wherein the speech test signal is configured for performing a speech audiometry for the user;
playing (842) the processed speech test signal and determining the speech audiometry result for the user using the hearing aid; and
in a case where the speech audiometry result indicates that a speech recognition rate of the user is less than a preset expected speech recognition rate, adjusting (844) the values of the gain of the hearing aid at different frequencies based on the speech audiometry result, and continuously iterating the speech audiometry based on the adjusted values of the gain until the speech recognition rate of the user is greater than or equal to the preset expected speech recognition rate.

5. The method as claimed in claim 4, wherein after the adjusting the values of the gain parameter of the hearing aid at different frequencies based on the sound-field test result until the hearing aid meets the preset first hear-aiding requirement, the method further comprises:
in a case where the hearing aid meets the preset first hear-aiding requirement, the hear-aiding thresholds at different frequencies for the user using the hearing aid are greater than or equal to the expected intensity values of the speech test signal at different frequencies respectively.

6. The method as claimed in claim 4, wherein the method further comprises:
in a case where the speech recognition rate of the user is greater than or equal to the preset expected speech recognition rate and the hear-aiding thresholds at different frequencies for the user using the hearing aid are greater than or equal to the expected intensity values of the speech test signal at different frequencies, guiding the user to undergo rehabilitation training.

7. The method as claimed in claim 4, wherein the speech audiometry result comprises speech recognition rates at different frequencies of the user using the hearing aid;
adjusting the values of the gain of the hearing aid at different frequencies based on the speech audiometry result, comprises:
extracting misrecognized words that the user has answered incorrectly from the speech audiometry result;
determining Ling's Six Sounds corresponding to the misrecognized words and a target frequency corresponding to the Ling's Six Sounds; and
adjusting a value of the gain of the hearing aid at the target frequency.

8. The method as claimed in any one of claims 1-7, wherein the hearing characteristic data comprises uncomfortable loudness levels at different frequencies and an audiogram in a preset time period, and the signal-processing parameters further comprise a maximum power output;
the initializing (200) the signal-processing parameters of the hearing aid at different frequencies based on the attribute data and the hearing characteristic data of the user, comprises:
determining (220) the values of the gain of the hearing aid at different frequencies based on the attribute data, the uncomfortable loudness levels at different frequencies, and the audiogram; and
initializing (220) the values of the gain and values of the maximum power output of the hearing aid at different frequencies.

9. The method as claimed in claim 8, wherein determining the uncomfortable loudness levels of the user at different frequencies, comprises:
playing a sound with a preset loudness under a specific frequency;
guiding the user to provide a feedback of whether the sound is tolerable;
in response to the feedback of the user that the sound is intolerable, determining the preset loudness as an uncomfortable threshold of the user under the specific frequency; and
in response to the feedback of the user that the sound is tolerable, increasing the loudness of the sound until the user indicates that the sound is intolerable, and determining a final loudness as the uncomfortable threshold of the user under the specific frequency.

10. The method as claimed in claim 8, wherein the initializing the maximum power output of the hearing aid at different frequencies, comprises:
determining the values of the maximum power output at different frequencies based on the uncomfortable loudness levels at different frequencies, and initializing the values of the maximum power output at different frequencies.

11. The method as claimed in claim 10, wherein the determining the maximum power output values of the hearing aid at different frequencies based on the uncomfortable loudness levels at different frequencies, comprises:
the values of the maximum power output of the hearing aid at different frequencies are not greater than the uncomfortable loudness levels of the user at different frequencies respectively.

12. The method as claimed in claim 7, wherein after the adjusting the values of the gain of the hearing aid at the target frequency, the method further comprises:
in a case where the number of gain adjustments reaches an upper limit, the speech audiometry result indicates that a speech recognition rate of the user at the target frequency is less than a preset expected speech recognition rate, and the sound-field test result indicates that the user has heard the pure-tone signal or the warble-tone signal at the target frequency, playing a preset rehabilitation training sample to conduct rehabilitation training for the user.

13. The method as claimed in any one of claims 1-7, wherein after continuously iteratively testing the hearing level of the user wearing the hearing aid based on the adjusted values of the gain, the method further comprises:
acquiring hearing test data fed back by the user; and
adjusting the values of the gain of the hearing aid at different frequencies based on the hearing test data until the hearing aid meets a preset second hear-aiding requirement.

14. The method as claimed in any one of claims 1-7, wherein after continuously iteratively testing the hearing level of the user wearing the hearing aid based on the adjusted values of the gain, the method further comprises:
acquiring hearing test data fed back by the user; and
adjusting the values of the gain of the hearing aid at different frequencies based on the hearing test data and the hearing characteristic data until the hearing aid meets a preset second hear-aiding requirement.

15. A hearing aid, comprising:
a Bluetooth module;
a speaker; and
a processor, connected to both the Bluetooth module and the speaker;
wherein the Bluetooth module is configured for receiving a hearing test signal at different frequencies and transmitting the received hearing test signal to the processor;
wherein the processor is configured for executing the method for fitting a hearing aid according to any one of claims 1 to 10 to adjust the values of the gain of the hearing aid at different frequencies, process the hearing test signal based on the adjusted values of the gain, and transmit the processed hearing test signal to the speaker;
wherein the speaker is configured for playing the processed hearing test signal.
